(19) Europäisches Patentamt
European Patent Office
Office européen des brevets

(11) Veröffentlichungsnummer: **0 320 726**
**A2**

(12) **EUROPÄISCHE PATENTANMELDUNG**

(21) Anmeldenummer: **88120206.3**

(22) Anmeldetag: **03.12.88**

(51) Int. Cl.⁴: **C07C 69/28 , C07C 67/20 , C07D 285/06**

(30) Priorität: **12.12.87 DE 3742222**

(43) Veröffentlichungstag der Anmeldung: **21.06.89 Patentblatt 89/25**

(84) Benannte Vertragsstaaten: **BE CH DE ES FR GB IT LI NL**

(71) Anmelder: **BASF Aktiengesellschaft**
**Carl-Bosch-Strasse 38**
**D-6700 Ludwigshafen(DE)**

(72) Erfinder: **Paul, Axel, Dr.**
**Kaefertaler Strasse 38**
**D-6800 Mannheim 1(DE)**
Erfinder: **Ladner, Wolfgang, Dr.**
**In den Bellen 5**
**D-6701 Fussgoenheim(DE)**
Erfinder: **Siegel, Hardo, Dr.**
**Hans-Purrmann-Allee 25**
**D-6720 Speyer(DE)**

(54) **(2R)-Aryloxy-3-n-butyroyloxy-propan-2-ole.**

(57) (2R)-Aryloxy-3-n-butyroyloxy-propan-2-ole der allgemeinen Formel I

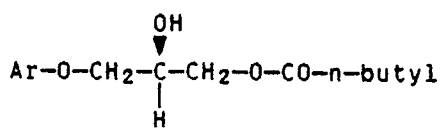

I

(Ar = isocyclischer oder heterocyclischer ein- oder mehrkernigen aromatischen Rest) sowie Verfahren zur Herstellung von I durch Umsetzung von (2R)-Glycidyl-n-butyrat (II) mit einer aromatischen Hydroxyverbindung Ar-OH (IV) in Gegenwart eines quartären Ammoniumhalogenids (III).

EP 0 320 726 A2

## (2R)-Aryloxy-3-n-butyroyloxy-propan-2-ole

Die vorliegende Erfindung betrifft (2R)-Aryloxy-3-n-butyroyloxy-propan-2-ole der allgemeinen Formel I

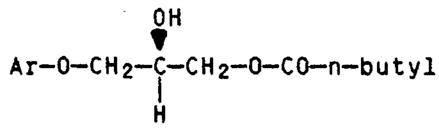

I

in der Ar einen isocyclischen oder heterocyclischen ein- oder mehrkernigen aromatischen Rest bedeutet, sowie ein Verfahren zur Herstellung dieser Verbindungen.

Aus Chemical & Engineering News, Juni 1986, Seite 24, ist es bekannt, das Herz- und Kreislaufmittel (2S)-Propranolol, ausgehend von (2R)-Glycidol und α-Naphthol, über die Zwischenverbindung (2R)-α-Naphthyloxypropan-2,3-diol herzustellen, wie das nachstehende Schema veranschaulicht:

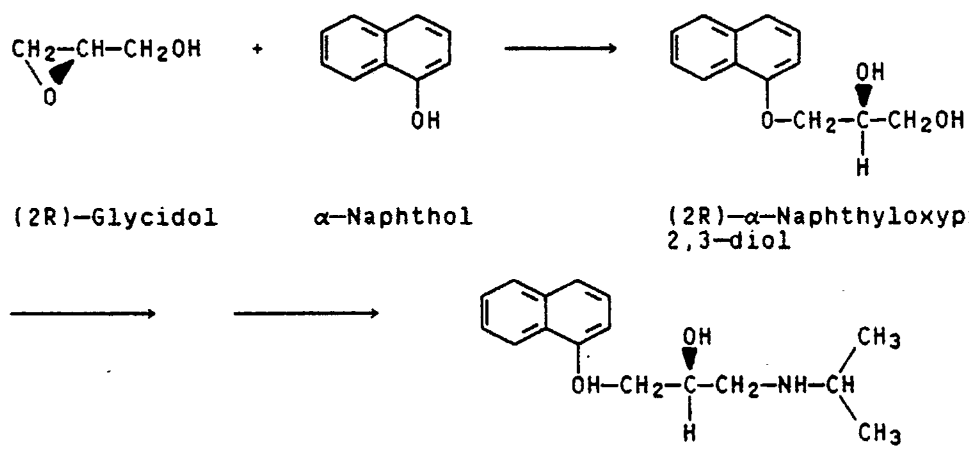

(2R)-Glycidol

α-Naphthol

(2R)-α-Naphthyloxypropan-2,3-diol

(2S)-Propranolol

Dieses Verfahren hat indes den Nachteil, daß das optisch aktive (2R)-Glycidol nur schwer zugänglich und entsprechend teuer ist.

Ferner ist es aus J. Am. Chem. Soc. Band 106 (1984), Seiten 7250-7251 bekannt, daß das (2R)-Glycidylbutyrat II

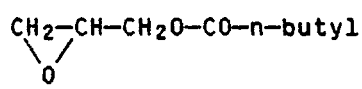

II

auf enzymatischem Wege relativ leicht erhältlich ist, so daß der Erfindung die allgemeine Aufgabe zugrunde lag, das (2S)-Propranolol und ähnliche Verbindungen mit dem Strukturelement I′

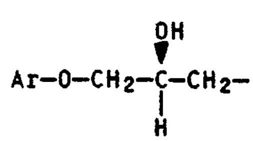

I′

unter Verwendung von (II) auf wirtschaftlichere Weise zugänglich zu machen als bisher. Speziell erstreckt sich die Aufgabe der Erfindung auf neue, für die Synthese von (2S)-Propranolol und ähnlichen Wirkstoffen geeignete Zwischenprodukte sowie auf ein Verfahren zur Herstellung dieser Zwischenprodukte.

Demgemäß wurden die eingangs definierten (2R)-Aryloxy-3-butyroyloxy-propan-2-ole I gefunden.

Außerdem wurde gefunden, daß man die Verbindungen I erhält, wenn man (2R)-Glycidyl-n-butyrat II in Gegenwart eines quartären Ammoniumhalogenids (III) mit einer aromatischen Hydroxyverbindung Ar-OH (IV) umsetzt.

Als quartäre Ammoniumhalogenide III, welche die Umsetzung von II mit IV katalytisch begünstigen, eignen sich praktisch alle Verbindungen dieses Typs, vornehmlich aber Jodide, gefolgt von den Bromiden und Chloriden. Bevorzugt werden Salze der Formel IIIa

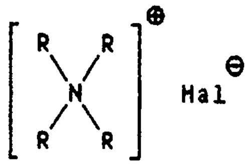

IIIa

in der die Reste R gleiche oder verschiedene Alkylgruppen mit 1 bis 4 C-Atomen bedeuten, wobei einer dieser Reste auch durch eine $C_5$ - $C_{18}$-Alkylgruppe oder die Benzylgruppe ersetzt sein kann und in der Hal Chlor, Brom oder Jod bedeutet.

Genannt seien beispielsweise Benzyltrimethylammoniumchlorid und -bromid, Tetramethylammoniumchlorid und -jodid sowie vor allem Tetra-(n-butyl)ammoniumbromid und -jodid.

Die Menge dieser Katalysatoren ist nicht kritisch, liegt jedoch zur Erzielung wirtschaftlich befriedigender Reaktionsgeschwindigkeiten in der Regel zwischen 0,5 und 20 mol% pro Mol IV.

Vorzugsweise läßt man II und IV im äquimolaren Mengenverhältnis miteinander reagieren, jedoch kann es im Falle sehr reaktionsträger Verbindungen IV zweckmäßig sein, eine dieser Komponenten im molaren Überschuß - etwa bis zu 50 mol% - einzusetzen.

Für die Reaktionstemperatur empfiehlt sich der Bereich von 50 bis 150 °C. Bei tieferen Temperaturen, etwa bis hinunter zu 0 °C, wird man normalerweise nur im Falle sehr empfindlicher Aryloxyverbindungen IV arbeiten, und bei höheren Temperaturen, insbesondere oberhalb 160 °C, ist mit Nebenreaktionen wie der Racemisierung von II und I und der Polymerisation von II zu rechnen.

In der Regel führt man die Umsetzung unter Normaldruck oder unter dem leicht erhöhten Eigendruck des Reaktionsgemisches aus, wenn man ein tief siedendes Lösungs- oder Verdünnungsmittel verwendet.

Die Mitverwendung eines Lösungs- oder Verdünnungsmittels ist prinzipiell nicht erforderlich, verfahrenstechnisch aber regelmäßig von Vorteil.

Als Lösungs- und Verdünnungsmittel - man kann sowohl in Lösung als auch in Dispersion arbeiten - eignen sich inerte organische Flüssigkeiten wie halogenierte Kohlenwasserstoffe, beispielsweise Methylenchlorid, Ether wie Diethylether oder Tetrahydrofuran, Ketone wie Aceton, Ester wie Ethylacetat und n-Butylacetat und aromatische Kohlenwasserstoffe wie Toluol. Die Menge der Lösungsmittel liegt im allgemeinen zwischen 1 und 6 kg pro kg IV.

Im übrigen bietet das erfindungsgemäße Verfahren keine verfahrenstechnischen Besonderheiten, so daß nähere Angaben hierzu entbehrlich sind. Das gleiche gilt für die Aufarbeitung der Reaktionsgemische.

Das gute Gelingen des erfindungsgemäßen Verfahrens ist von der Art der Reste Ar nicht erkennbar abhängig, sofern sie keine nicht-inerten Substituenten, wie Carboxyl-, Sulfo-, Thiol-, Hydroxy- oder primäre oder sekundäre Aminogruppen tragen.

Die aromatischen Reste Ar können sich beispielsweise vom Benzol, dem Naphthalin, dem Pyridin, dem Pyrimidin, dem Pyrazin, den Triazolen, dem Chinolin, dem Oxazol, dem Isoxazol, den Thiaoxazolen und -dioxazolen, dem Indol, dem Carbazol, dem Furan und dem Thiophen ableiten, wobei die Hydroxylgruppe an beliebiger Ringposition stehen kann.

Diese aromatischen Stammkörper können ein oder mehrere inerte Substituenten wie $C_1$-$C_4$-Alkylgruppen, $C_1$-$C_4$-Alkoxygruppen, $C_1$-$C_4$-Carbalkoxygruppen, $C_2$-$C_4$-Alkoxycarbonylgruppen, $C_2$-$C_4$-Acylgruppen und die entsprechenden olefinisch oder acetylenisch ungesättigten Gruppen mit jeweils mindestens zwei bzw. drei C-Atomen tragen sowie des weiteren Cycloalkyl-und Cycloalkenylreste mit 3 bis 7 Ringgliedern, araliphatische Gruppen wie die Benzylgruppe oder die 1- oder 2-Phenylethyl- oder Ethenylgruppe, aromatische Reste wie die Phenylgruppe, Halogen wie Fluor, Chlor oder Brom, Halogenalkylgruppen wie die Trifluormethylgruppe, Dialkylaminogruppen mit insgesamt 2 bis 6 C-Atomen, die Cyangruppe und die Nitrogruppe.

Besondere Bedeutung haben das erfindungsgemäße Verfahren und damit das entsprechende Zwischenprodukt I im Hinblick auf das bekannte Arzneimittel (2R)-Propranolol für den Fall der $\alpha$-Naphthylgruppe als Arylgruppe Ar.

Ebenso verhält es sich mit den aromatischen Hydroxyverbindungen

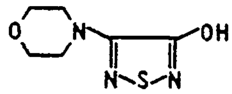

3-Hydroxy-4-(1-morpholino)-1,2,5-thiadiazol

und

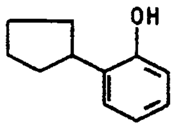

2-Cyclopentylphenol

von denen sich die Arzneimittel Timolol und Penbutolol ableiten.

Allgemein erweitern das erfindungsgemäße Verfahren und die dazugehörigen Verbindungen I die Möglichkeit zur wirtschaftlichen Synthese zahlreicher Verbindungen auf dem Gebiet der wichtigen Wirkstoffe mit dem Strukturelement I'.

Bei der weiteren Umsetzung von I wird man in der Regel zunächst die n-Butyroyloxygruppe in an sich bekannter Weise hydrolytisch zum entsprechenden Propandiolderivat abspalten und dieses dann den Folgereaktionen unterwerfen.

Beispiele 1

Herstellung von (2R)-α-Naphthyloxy-3-n-butyroyloxypropan-2-ol

Eine Lösung aus 200 ml Toluol, 144 g (1 mol) α-Naphthol und 7,4 g (0,02 mol) wurde im Laufe von 15 min bei 120° C mit 158 g (1 mol) (2R)-Glycidylbutyrat versetzt und 4 h bei dieser Temperatur gehalten. Die übliche Aufarbeitung des Reaktionsgemisches lieferte die oben genannte Verbindung in Form eines Öles in einer Ausbeute von rd. 75%.

NMR ($CDCl_3$) t 0,92 (3H), J = 7; sextett 1,65 (2H), J = 7; t 2,33 (2H), J = 7; m 4,20 (2H); m 4,30 (3H); d 6,78 (1H), J = 7; dd 7,34 (1H), J = 7+7; m 7,4 (4H); m 7,8 (1H); m 8,2 (1H)

Beispiel 2

Herstellung von (2R)-2-Cyclopentylphenoxy-3-n-butyroyloxy-propan-2-ol

Diese Verbindung, die ebenfalls in Form eines Öles anfiel, wurde in 72%iger Ausbeute analog Beispiel 1 aus 162 g (1 mol) 2-Cyclopentylphenol und den gleichen Mengen der dort angegebenen übrigen Reaktionsteilnehmer hergestellt.

NMR ($CDCl_3$) t 0,95 (3H), J = 7; m 1,7 (6H); sextett 1,67 (2H), J = 7; m 2,0 (2H); t 2,38 (2H), J = 7; 2,73 sbr. (1H); quintett 3,33 (1H), J = 7; m 4,05 (2H); m 4,30 (3H); d 6,83 (1H), J = 7; t 6,95 (1H), J = 7; t 7,16 (1H); J = 7; d 7,23 (1H); J = 7

## Ansprüche

1. (2R)-Aryloxy-3-n-butyroyloxy-propan-2-ole der allgemeinen Formel I

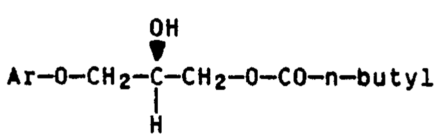

I

in der Ar einen isocyclischen oder heterocyclischen ein- oder mehrkernigen aromatischen Rest bedeutet.

2. α-Naphthyloxy-3-n-butyroyloxypropan-2-ol

3. (2R)-3-[4-(1-Morpholino)-1,2,5-thiadiazol-3-yl]-oxy-3-n-butyroyloxy-propan-2-ol

4. (2R)-3-(2-Cyclopentyl)-phenoxy-3-n-butyroyloxy-propan-2-ol

5. Verfahren zur Herstellung von (2R)-Aryloxy-3-n-butyroyloxy-propan-2-olen (I) gemäß Anspruch 1, dadurch gekennzeichnet, daß man (2R)-Glycidyl-n-butyrat (II) in Gegenwart eines quartären Ammoniumhalogenids (III) mit einer aromatischen Hydroxyverbindung Ar-OH (IV) umsetzt.

6. Verfahren nach Anspruch 5, dadurch gekennzeichnet, daß man als quartäres Ammoniumhalogenid III ein Jodid verwendet.

7. Verfahren nach den Ansprüche 5 und 6, dadurch gekennzeichnet, daß man als quartäres Ammoniumhalogenid III ein Salz der Formel IIIa

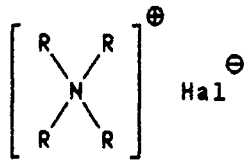

IIIa

verwendet, in der die Reste R gleiche oder verschiedene Alkylgruppen mit 1 bis 4 C-Atomen bedeuten, wobei einer dieser Reste durch eine $C_5$ - $C_{18}$-Alkylgruppe oder die Benzylgruppe ersetzt sein kann, und in der Hal Chlor, Brom oder Jod bedeutet.